Europäisches Patentamt

European Patent Office

Office européen des brevets

(19)

(11) **EP 0 766 541 B1**

(12) **FASCICULE DE BREVET EUROPEEN**

(45) Date de publication et mention
de la délivrance du brevet:
**28.04.2004 Bulletin 2004/18**

(51) Int Cl.⁷: **A61F 2/38**

(21) Numéro de dépôt: **95923423.8**

(22) Date de dépôt: **21.06.1995**

(86) Numéro de dépôt international:
**PCT/FR1995/000823**

(87) Numéro de publication internationale:
**WO 1995/035074 (28.12.1995 Gazette 1995/55)**

(54) **IMPLANT FEMORAL NOTAMMENT POUR PROTHESE TRICOMPARTIMENTALE DU GENOU**

FEMURIMPLANTAT, INSBESONDERE FÜR EINE DREIGETEILTE KNIEPROTHESE

FEMORAL IMPLANT PARTICULARLY FOR A TRICOMPARTMENTAL PROSTHESIS OF THE KNEE

(84) Etats contractants désignés:
**DE ES GB IT**

(30) Priorité: **22.06.1994 FR 9407964**

(43) Date de publication de la demande:
**09.04.1997 Bulletin 1997/15**

(73) Titulaire: **EUROS Société Anonyme
F-13600 La Ciotat (FR)**

(72) Inventeurs:
• **O'ZOUX, Patrick
F-21200 Beaune (FR)**
• **CHAIX, Claude
F-13008 Marseille (FR)**
• **MOUTTET, Alexandre
F-66300 Thuir (FR)**
• **CALAS, Philippe
F-13100 Aix-en-Provence (FR)**
• **NERKOWSKI, Edwin
F-13790 Châteauneuf-Lerouge (FR)**

(74) Mandataire: **Somnier, Jean-Louis et al
Novagraaf Technologies
122, rue Edouard Vaillant
92593 Levallois Perret Cedex (FR)**

(56) Documents cités:
**EP-A- 0 021 421          WO-A-93/05729
US-A- 4 822 365**

EP 0 766 541 B1

**Description**

D'une manière parfaitement connue, un implant fémoral comprend des patins condyliens interne et externe, susceptibles d'être reliés, du côté antérieur, par une trochlée sur laquelle peut coopérer en appui un bouton rotulien. Les patins condyliens sont destinés à coopérer, en appui et à glissement, sur des surfaces profilées ou non, que présente un implant tibial.

Les patins condyliens fémoraux peuvent présenter différents profils. Généralement, les différents profils proposés à ce jour, sont déterminés pour se rapprocher le plus possible de l'anatomie naturelle de la tête fémorale. On peut citer par exemple, l'enseignement du brevet EP 0021421 qui définit une prothèse du genou comprenant un implant fémoral et un implant tibial, avec interposition d'un élément intermédiaire entre lesdits implants.

Le problème que se propose de résoudre l'invention, est de déterminer les profils des patins condyliens interne et externe, par une description cinématique du mouvement relatif du fémur par rapport au tibia, en ayant pour objectif de créer des profils compatibles avec la cinématique réelle d'un genou naturel.

Un autre problème que se propose de résoudre l'invention, est de tenir compte des différents éléments de l'anatomie réelle inclus dans la prothèse, à savoir notamment, la forme particulière des implants tibiaux, qui peut varier en fonction du cas pathologique à traiter.

Un autre problème que se propose de résoudre l'invention, est d'avoir des profils répondant à l'angle de varus valgus dynamique, et du déplacement axial qui existe dans la cinématique réelle d'un genou naturel.

Pour résoudre de tels problèmes, il a été conçu et mis au point un implant fémoral notamment pour prothèse tricompartimentale du genou comprenant des patins condyliens reunis du côté antérieur, par une trochlée , les patins condyliens étant destinés à être en contact direct avec un implant tibial.

L'invention concerne un implant fémoral selon la revendication 1 ou 2.

L'invention est exposée, ci-après plus en détail à l'aide des dessins annexés, dans lesquels :

La figure 1 est une vue de côté montrant les profils postérieurs de base.

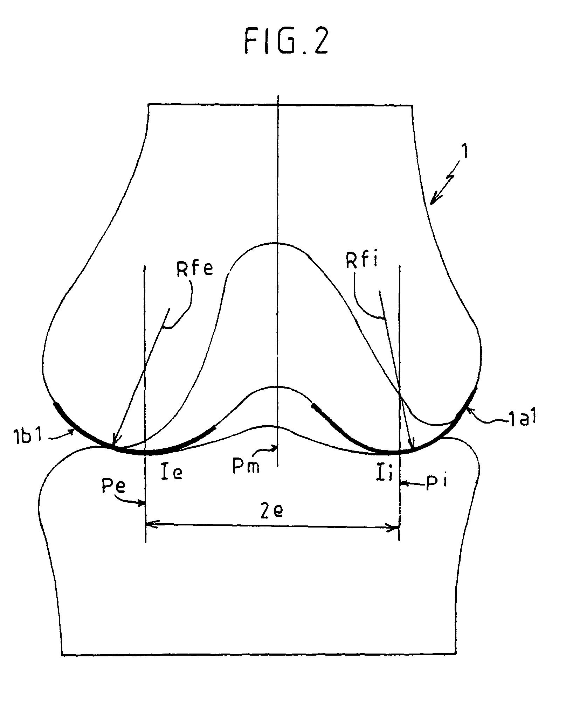

La figure 2 est une vue de face correspondant à la figure 1.

Les figures 3 et 4 sont des diagrammes montrant les profils postérieur et antérieur, cinématiquement admissibles, d'une part, des patins condyliens et, d'autre part, de la trochlée.

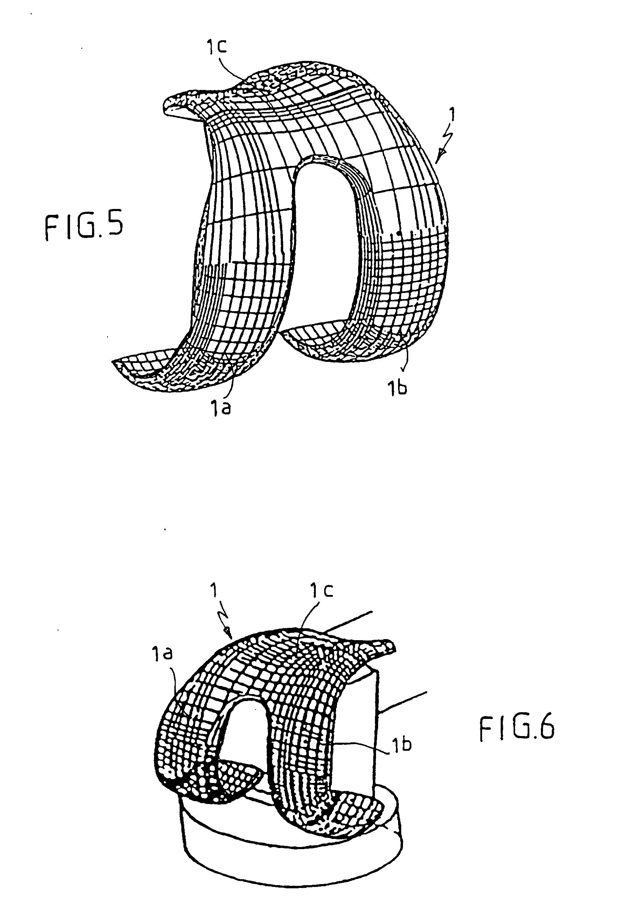

Les figures 5 et 6 sont des vues en perspective de l'implant fémoral dissymétrique selon l'invention.

L'implant fémoral est désigné dans son ensemble par (1), tandis que les patins condyliens interne et externe sont respectivement désignés par (1a) et (1b). La trochlée est désignée par (1c).

Selon l'invention, les profils postérieurs des patins interne (1a) et externe (1b) sont dissymétriques sans être des copies anatomiques de la tête fémorale.

Les profils postérieurs sont construits sur la base de deux profils identiques pour les condyles externe et interne. La description et la simulation numérique du mouvement relatif du fémur par rapport au tiba, selon les trois plans de l'espace, doit tenir compte :

- d'une part, des trois rotations relatives, à savoir :

  * une flexion d'angle ($\alpha$),
  * une rotation axial automatique Ry($\alpha$),
  * une rotation valgus varus Vz($\alpha$) pour un genou sain, aux formes réelles des condyles et des plateaux tibiaux.

- d'autre part, des trois translations relatives, à savoir :

  * latéro-médiane Dx($\alpha$),
  * axiale Dy($\alpha$) résultant des formes réelles des condyles et des plateaux tibiaux,
  * antéro-postérieure Dz($\alpha$) résultant du glissement.

Ces différentes dispositions nécessitent d'ajuster les profils de base, pour chaque angle de flexion. Ce profil géométrique de base est constitué par deux sections toriques complexes et identiques (1a1) (1b1), situées dans deux plans parallèles interne (Pi) et externe (Pe) et à égale distance du plan sagittal moyen (Pm).

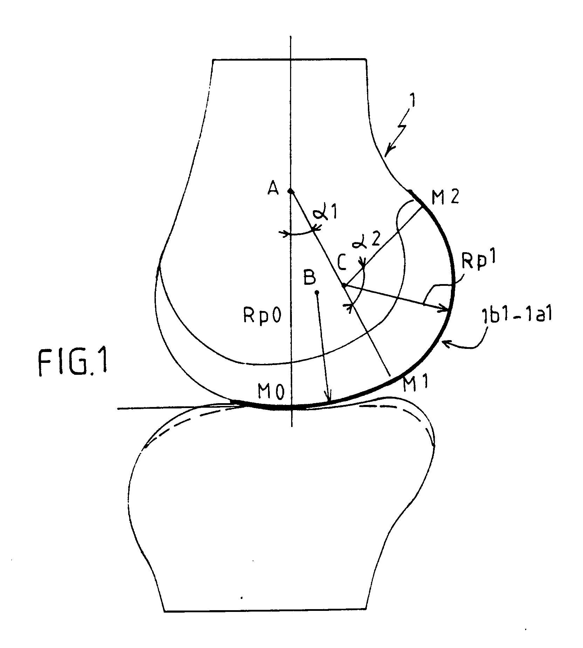

Plus particulièrement, comme le montre la figure 1, dans le plan sagittal, les sections toriques (1b1) sont constituées par des zones postérieures (M1) (M2), assimilables à des sphères de centre (C) et de rayon (Rp1) qui correspond au rayon postérieur moyen en flexion.

En outre, les profils de raccordement des sections toriques, sont caractérisés par :

- l'écartement (2e) des deux plans externe (Pe) et interne (Pi),
- le rayon postérieur moyen en extension (Rp0),
- le rayon postérieur moyen en flexion (Rp1),
- l'angle ($\alpha$1) de l'arc primaire (M0-M1),
- l'angle ($\alpha$2) de l'arc secondaire précité (M1-M2),

A noter que les rayons de courbure (Rfe) et (Rfi) des sections toriques (1b1) (1a1), selon une coupe frontale, sont constants.

En ce qui concerne la forme des profils de base des patins condyliens interne et externe considérés dans un plan sagittal, il convient de distinguer l'arc primaire (M0-M1) et l'arc secondaire (M1-M2).

Lorsque l'angle de flexion ($\alpha$) est inférieur à ($\alpha$1), les contacts (Ie) et (Ii), évoluent dans la zone (M0-M1). Les rayons (AB) sont constants et égaux à (Rp0) - (Rp1). Seuls les rayons (VM) sont calculés et ajustés pour répondre aux impératifs cinématiques. Les distances (BI) et (BM) sont égales en externe comme en interne, les rayons de courbure de sections frontales étant constants.

Dans ces conditions, le rayon de courbure sagittal du profil du patin condylien externe (1b) est égal à :

$$Rp0 - (Rp0\text{-}Rp1) \cdot ((1\text{-}\cos (\alpha + \alpha1) \cdot \cos (Vz (\alpha)) + \cos (\alpha1)) + Dy (\alpha) - e.\sin (Vz (\alpha)),$$

tandis que le rayon de courbure sagittal du profil du patin condylien interne (1a) est égal à :

$$Rp0 - (Rp0\text{-}Rp1) \cdot ( (1\text{-}\cos (\alpha + \alpha1) \cdot \cos (Vz (\alpha)) + \cos (\alpha1)) + Dy (\alpha) + e.\sin (Vz (\alpha)).$$

Ces différentes valeurs s'ajoutent au rayon (AB) pour donner les rayons de courbure sagittaux des profils externe et interne pour la zone (M0-M1).

Lorsque l'angle de flexion ($\alpha$) est supérieur à l'angle ($\alpha$1) et inférieur à l'angle ($\alpha$2) de l'arc secondaire (M1-M2), les contacts (Ie) et (Ii) évoluent dans la zone (M1-M2). Soit (CM) les rayons de courbure correspondants. Les distances (CI) et (CM) sont égales en externe comme en interne, les rayons de courbure des sections frontales étant constants.

Dans ces conditions, le rayon de courbure sagittal du profil du patin condylien externe (1b) est égal à :

$$Rp0 - (Rp0\text{-}Rp1) \cdot \cos(\alpha\ 1) + Dy(\alpha) - e.\sin(Vz(\alpha)).$$

tandis que le rayon de courbure sagittal du profil condylien interne (1a) est égal à :

$$Rp0 - (Rp0\text{-}Rp1) \cdot \cos(\alpha1) + Dy(\alpha) + e.\sin(Vz(\alpha)).$$

Dans ces différentes formules :

Rp0 est le rayon postérieur moyen en extension,
Rp1 est le rayon postérieur moyen en flexion,
$Vz(\alpha)$ est la rotation de valgus/varus,
$Dy (\alpha)$ est la translation axiale.

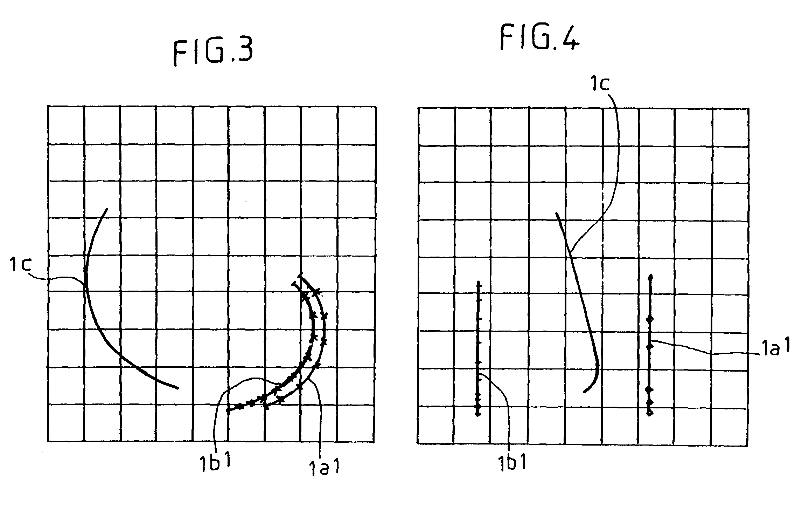

Il en résulte des profils postérieurs externe et interne différents, comme le montre la figure 3 et par conséquent une partie postérieure de prothèse dissymétrique, en étant cinématiquement admissible. A noter que cette figure montre le résultat obtenu dans le cas d'une simulation avec plateau tibial plat.

L'invention s'applique toutefois à toutes formes de plateaux et à toutes formes de profils fémoraux de base, la dissymétrie étant simplement modifiée.

En ce qui concerne le profil de la gorge trochléenne (1c), cette dernière est profilée en fonction des diverses positions de la rotule par rapport au fémur, notamment en tenant compte des mouvements relatifs rotule-tibia puis tibia-fémur.

Le profil obtenu est montré aux figures 3 et 4.

Les avantages ressortent bien de la description.

**Revendications**

1. Implant fémoral, notamment pour prothèse tri-compartimentalc du genou, comprenant des profils postérieurs externe (1b1) et interne (1a1) de patins condyliens (1a, 1b), les patins condyliens étant réunis du côté antérieur par une trochlée (1c), ces patins étant destinés à être en contact direct avec un implant tibial, les profils postérieurs externe (1b1) et interne (1a1) des patins étant constitués, dans un plan frontal, deux sections en forme de tores complexes externe et interne situées à égale distance du plan sagittal moyen (Pm), les rayons de courbure (Rfe, Rfi) desdites sections dans le plan frontal étant constants et égaux au rayon postérieur moyen en flexion (Rp1), pour obtenir des zones postérieurs assimilables à des sphères,

   **caractérisé en ce que**:

   - les profils (1b1,1a1) des sections de tores complexes présentent deux zones distinctes dont l'une correspond à une zone d'angle de flexion inférieure à un premier angle déterminé ($\alpha$1) d'un arc primaire (M0-M1), l'autre zone correspondant à une zone d'angle de flexion supérieur audit premier angle ($\alpha$1), mais inférieur à un second angle déterminé ($\alpha$2) d'un arc secondaire (M1-M2),
   - dans la zone d'arc primaire (M0-M1) où l'angle de flexion est inférieur audit premier angle ($\alpha$1),
   - le rayon de courbure sagittal du profil du patin condylien externe (1 b) est égal à :

   $$Rp0 - (Rp0-Rp1) \cdot ((1-\cos(\alpha+\alpha1) \cdot \cos(Vz(\alpha)) + \cos(\alpha1)) + Dy(\alpha) - e.\sin(Vz(\alpha)).$$

   - le rayon de courbure sagittal du profil du patin condylien interne (1a) est égal à :

   $$Rp0 - (Rp0-Rp1) \cdot ((1-\cos(\alpha+\alpha1) \cdot \cos(Vz(\alpha)) + \cos(\alpha1)) + Dy(\alpha) + e.\sin(Vz(\alpha)),$$

   formule dans laquelle :

   Rp0 est le rayon postérieur moyen en extension,
   Rp1 est le rayon postérieur moyen en flexion,
   Vz($\alpha$) est la rotation de valgus/varus,
   Dy($\alpha$) est la translation axiale.

2. Implant fémoral, notamment pour prothèse tricompartimentale du genou, comprenant des profils postérieurs externe (1b1) et interne (1a1) de patins condyliens (1a, 1b), les patins condyliens étant réunis du côté antérieur par une trochlée (1c), ces patins étant destinés à être en contact direct avec un implant tibial, les profils postérieurs externe (1b1) et interne (1a1) des patins étant constitués, dans un plan frontal, deux sections en forme de tores complexes externe et interne situées à égale distance du plan sagittal noyen (Pm), les rayons de courbure (Rfe, Rfi) desdites seccions dans le plan frontal étant constants et égaux au rayon postérieur moyen en flexion (Rp1), pour obtenir des zones postérieurs assimilables à des sphères,

   **caractérisé en ce que**:

   - les profils (1b1,1a1) des sections de tores complexes présentent deux zones distinctes dont l'une correspond à une zone d'angle de flexion inférieure à un premier angle déterminé ($\alpha$1) d'un arc primaire (M0-M1), l'autre zone correspondant à une zone d'angle de flexion supérieur audit premier angle ($\alpha$1), mais inférieur à un second angle déterminé ($\alpha$2) d'un arc secondaire (M1-M2),
   - et, dans la zone d'arc secondaire (M1-M2) où l'angle de flexion ($\alpha$) est supérieur audit premier angle prédéterminé ($\alpha$1) :

   • le rayon de courbure sagittal du profil du patin condylien externe (1b) est égal à :

   $$Rp0-(Rp0-Rp1).\cos(\alpha1)+Dy(\alpha)-e.\sin(Vz(\alpha)),$$

   • et le rayon de courbure sagittal du profil du patin condylien interne (la) est égal à :

   $$Rp0-(Rp0-Rp1) \cdot \cos(\alpha1)+Dy(\alpha)+e.\sin(Vz(\alpha)),$$

formules dans lesquelles :

Rp0 est le rayon postérieur moyen en extension,
Rp1 est le rayon postérieur moyen en flexion,
Vz($\alpha$) est la rotation de valgus/varus,
Dy($\alpha$) est la translation axiale.

**Claims**

1. Femoral implant, in particular for a tricompartmental prosthesis of the knee, comprising external (1b1) and internal (1a1) posterior profiles of condylar plates (1a, 1b), the condylar plates being joined on the anterior side by a trochlea (1c), these plates being intended to be in direct contact with a tibial implant, the external (1b1) and internal (1a1) external posterior profiles of the plates consisting, in a frontal plane, of two sections in the form of complex external and internal toruses situated at equal distances from the mean sagittal plane (Pm), the radii of curvature (Rfe, Rfi) of the said sections in the frontal plane being constant and equal to the mean posterior radius in flexion (Rp1), in order to obtain posterior zones which can be assimilated to spheres, **characterised in that**:

   - the profiles (1b1, 1a1) of the sections of complex toruses have two distinct zones, one of which corresponds to a zone with a flexion angle less than a first determined angle ($\alpha$1) of a primary arc (M0-M1), the other zone corresponding to a zone with a flexion angle greater than the said first angle ($\alpha$1) but less than a second determined angle ($\alpha$2) of a secondary arc (M1-M2),

   - in the primary arc zone (M0-M1) where the flexion angle is less than the said first angle ($\alpha$1),

   - the sagittal radius of curvature of the profile of the external condylar plate (1b) is equal to:

$$Rp0-(Rp0-Rp1) . ((1-\cos (\alpha+\alpha1) .\cos(Vz(\alpha))+\cos(\alpha1))+Dy(\alpha)-$$

$$e.\sin(Vz(\alpha)),$$

   - the sagittal radius of curvature of the profile of the internal condylar plate (1a) is equal to:

$$Rp0-(Rp0-Rp1) . ((1-$$

$$\cos(\alpha+\alpha1).\cos(Vz(\alpha))+\cos(\alpha1))+Dy(\alpha)+e.\sin(Vz(\alpha)),$$

   a formula in which:

   Rp0 is the mean posterior radius in extension,

   Rp1 is the mean posterior radius in flexion,

   Vz($\alpha$) is the valgus/varus rotation,

   Dy($\alpha$) is the axial translation.

2. Femoral implant, in particular for a tricompartmental prosthesis of the knee, comprising external (1b1) and internal (1a1) posterior profiles of condylar plates (1a, 1b), the condylar plates being joined on the anterior side by a trochlea (1c), these plates being intended to be in direct contact with a tibial implant, the external (1b1) and internal (1a1) external posterior profiles of the plates consisting, in a frontal plane, of two sections in the form of complex external and internal toruses situated at equal distances from the mean sagittal plane (Pm), the radii of curvature (Rfe, Rfi) of the said sections in the frontal plane being constant and equal to the mean posterior radius in flexion (Rp1), in order to obtain posterior zones which can be assimilated to spheres, **characterised in that**:

- the profiles (1b1, 1a1) of the sections of complex toruses have two distinct zones, one of which corresponds to a zone with a flexion angle less than a first determined angle ($\alpha$1) of a primary arc (M0-M1), the other zone corresponding to a zone with a flexion angle greater than the said first angle ($\alpha$1) but less than a second determined angle ($\alpha$2) of a secondary arc (M1-M2),

- and, in the secondary arc zone (M0-M1) where the flexion angle is less than the said first predetermined angle ($\alpha$1),

• the sagittal radius of curvature of the profile of the external condylar plate (1b) is equal to:

$$Rp0-(Rp0-Rp1).\cos(\alpha1)+Dy(\alpha)-e.\sin(Vz(\alpha)),$$

• the sagittal radius of curvature of the profile of the internal condylar plate (1a) is equal to:

$$Rp0-(Rp0-Rp1).\cos(\alpha1)+Dy(\alpha)+e.\sin(Vz(\alpha)),$$

formulae in which:

Rp0 is the mean posterior radius in extension,

Rp1 is the mean posterior radius in flexion,

Vz($\alpha$) is the valgus/varus rotation,

Dy($\alpha$) is the axial translation.

**Patentansprüche**

1. Femurimplantat, insbesondere für eine dreigeteilte Knieprothese mit einem hinteren Außenprofil (1b1) und einem hinteren Innenprofil (1a1) der Gelenkkopfkufen (1a, 1b), wobei die Gelenkkopfkufen an der Vorderseite durch eine Trochlea (1c) vereinigt sind, wobei diese Kufen dazu bestimmt sind, in direktem Kontakt mit einem Schienenbeinimplantat zu stehen, wobei das hintere Außenprofil (1b1) und das hintere Innenprofil (1a1) der Kufen in einer vorderen Ebene durch zwei Abschnitte in Form von einer äußeren und einer inneren komplexen Ringfläche gebildet sind, die sich in gleichem Abstand zur mittleren Sagittalebene (Pm) befinden, wobei die Krümmungsradien (Rfe; Rfi) der Abschnitte in der vorderen Ebene konstant sind und gleich dem hinteren mittleren Beugungsradius (Rp) sind, um hintere Bereiche, die Kugeln ähnlich sind, zu erhalten,
**dadurch gekennzeichnet, dass**
die Profile (1b1; 1a1) der Abschnitte mit komplexen Ringflächen zwei unterschiedliche Bereiche bilden, von denen einer einem Beugungswinkelbereich entspricht, der kleiner als ein vorbestimmter erster Winkel ($\alpha$1) eines Primärbogen (M0-M1) ist, und wobei der andere Bereich einem Beugungswinkelbereich entspricht, der größer als der erste Winkel ($\alpha$1) jedoch kleiner als ein vorbestimmter zweiter Winkel ($\alpha$2) eines Sekundärbogens (M1-M2) ist,
im Bereich vom Primärbogen (M0-M1), wo der Beugungswinkel kleiner als der erste Winkel ($\alpha$1) ist, der Sagittalkrümmungsradius des Profils der äußeren Gelenkkopfkufe (1b) gleich

$$Rp0 - (Rp0 - Rp1) \cdot ((1 - \cos(\alpha + \alpha1) \cdot \cos(Vz(\alpha)) + \cos(\alpha1)) + Dy(\alpha) - e \cdot \sin(Vz(\alpha))$$

ist,
der Sagittalkrümmungsradius vom Profil der inneren Gelenkkopfkufe (1a) gleich

$$Rp0 - (Rp0 - Rp1) \cdot ((1- \cos(\alpha + \alpha1) \cdot \cos(Vz(\alpha)) + \cos(\alpha1)) + Dy(\alpha) + e \cdot \sin(Vz(\alpha))$$

ist,
wobei in den Formeln

Rp0 der hintere mittlere Streckungsradius ist,
Rp1 der hintere mittlere Beugungsradius ist,
Vz($\alpha$) die Valgus/Varus-Rotation ist,
Dy($\alpha$) die Axialverschiebung ist.

2. Femurimplantat, insbesondere für eine dreigeteilte Knieprothese mit einem hinteren Außenprofil (1b1) und einem hinteren Innenprofil (1a1) der Gelenkkopfkufen (la, 1b), wobei die Gelenkkopfkufen an der Vorderseite durch eine Trochlea (1c) vereinigt sind, wobei diese Kufen dazu bestimmt sind, in direktem Kontakt mit einem Schienenbeinimplantat zu stehen, wobei das hintere Außenprofil (1b1) und das hintere Innenprofil (1a1) der Kufen in einer vorderen Ebene durch zwei Abschnitte in Form von einer äußeren und einer inneren komplexen Ringfläche gebildet sind, die sich in gleichem Abstand zur mittleren Sagittalebene (Pm) befinden, wobei die Krümmungsradien (Rfe; Rfi) der Abschnitte in der vorderen Ebene konstant sind und gleich dem hinteren mittleren Beugungsradius (Rp1) sind, um hintere Bereiche, die Kugeln ähnlich sind, zu erhalten,
**dadurch gekennzeichnet, dass**
die Profile (1b1; 1a1) der Abschnitte mit komplexen Ringflächen zwei unterschiedliche Bereiche bilden, von denen einer einem Beugungswinkelbereich entspricht, der kleiner als ein vorbestimmter erster Winkel ($\alpha$1) eines Primär-bogen (M0-M1) ist, und wobei der andere Bereich einem Beugungswinkelbereich entspricht, der größer als der erste Winkel ($\alpha$1) jedoch kleiner als ein vorbestimmter zweiter Winkel ($\alpha$2) eines Sekundärbogens (M1-M2) ist, und im Bereich des Sekundärbogens (M1-M2), wo der Beugungswinkel ($\alpha$) größer als der vorbestimmte erste Winkel ($\alpha$1) ist,

- der Sagittal-Krümmungsradius vom Profil der äußeren Gelenkkopfkufe (1b) gleich

$$Rp0 - (Rp0 - Rp1) \cdot \cos(\alpha1) + Dy(\alpha) - e \cdot \sin(Vz(\alpha))$$

ist,
- und der Sagittal-Krümmungsradius vom Profil der inneren Gelenkkopfkufe (la) gleich

$$Rp0 - (Rp0 - Rp1) \cdot \cos(\alpha1) + Dy(\alpha) + e \cdot \sin(Vz(\alpha))$$

ist,

wobei in den Formeln
Rp0 der hintere mittlere Streckungsradius ist,
Rp1 der hintere mittlere Beugungsradius ist,
Vz($\alpha$) die Valgus/Varus-Rotation ist,
Dy($\alpha$) die Axialverschiebung ist.

# FIG.1

# FIG.2

FIG.3

FIG.4

FIG.5

FIG.6